(19) Europäisches Patentamt
European Patent Office
Office européen des brevets

(11) Publication number: 0 336 155 A1

(12) # EUROPEAN PATENT APPLICATION

(21) Application number: 89104607.0

(22) Date of filing: 15.03.89

(51) Int. Cl.4: C12N 15/00 , C12P 21/02 , C07K 13/00

(30) Priority: 17.03.88 JP 64142/88

(43) Date of publication of application: 11.10.89 Bulletin 89/41

(84) Designated Contracting States: DE FR GB IT

(71) Applicant: Yamasa Shoyu Kabushiki Kaisha
10-1, Araoi-Cho 2-Chome
Choshi-Shi Chiba-Ken 288(JP)

(72) Inventor: Kurabayashi, Masahiko
17-5-801, Hongo 2-Chome Bunkyo-Ku
Tokyo-To(JP)
Inventor: Yazaki, Yoshio
12-4, Kobinata 3-Chome Bunkyo-Ku
Tokyo-To(JP)
Inventor: Noguchi, Toshitada
4399-6, Nishi-Ogawa-Cho
Choshi-Shi Chiba-Ken(JP)
Inventor: Ohtani, Yutaka
2-1, Araoi-Cho 2-Chome
Choshi-Shi Chiba-Ken(JP)
Inventor: Kato, Hirohisa
2-2, Sakae-Cho, 2-Chome
Choshi-Shi Chiba-Ken(JP)
Inventor: Sugi, Masahito
2-2, Sakae-Cho, 2-Chome
Choshi-Shi Chiba-Ken(JP)

(74) Representative: Dr. Elisabeth Jung Dr. Jürgen Schirdewahn Dipl.-Ing. Claus Gernhardt
P.O. Box 40 14 68 Clemensstrasse 30
D-8000 München 40(DE)

(54) **Recombinant human cardiac myosin.**

(57) Substantially pure recombinant human cardiac myosin protein and fragments thereof free from other proteins of human origin are disclosed. The production and use of such protein is also disclosed.

EP 0 336 155 A1

Xerox Copy Centre

# RECOMBINANT HUMAN CARDIAC MYOSIN

## BACKGROUND OF THE INVENTION

The present invention relates to a recombinant human cardiac myosin protein, to a DNA sequence coding for said protein, to a process for the production of said recombinant human cardiac myosin, to an expression vector to be used in said process and to a host cell transformed by said expression vector.

The human cardiac myosin is a structural protein with a molecular weight of about 500,000 consisting of subunits comprising two heavy chains with a molecular weight of 200,000, two light chains I (LI) with a molecular weight of 27,000 and two light chains II (LII) with a molecular weight of 20,000. Furthermore, it exists abundantly (about 60%) among myocardial cell constituting proteins and plays a leading role in the muscle contraction mechanism.

The cardiac myosin, especially the cardiac myosin light chain or a fragment thereof, is released from myocardial cells into the blood as a consequence of destruction of cell membranes due to ischemic cardiac disorders. Accordingly, the cardiac myosin is considered to directly reflect the destruction process of myocardial cell due to ischemia. Therefore, the measurement of the concentration of cardiac myosin, especially the cardiac myosin light chain or a fragment thereof in the blood is considered to be clinically significant as a specific means for diagnosing myocardial infarction (Jap. J. Internal Med., 70, 16 (1981)).

Reported methods for the measurement of the cardiac myosin concentration in the blood include immunological assays such as those by the use of antiserum specific to the light chain of human cardiac myosin (Am. J. Cardiol., 41, 641 (1987); Circulation, 58, 1130 (1979); Circulation, 60, 139 (1979)) and those by the use of the monoclonal antibody specific to the light chain of cardiac myosin (J. Mol. Cell Cardiol., 14, 139 (1982); Molecular Immunology, 14, 451 (1982); Japanese Patent Laid-Open Pub. No. 286752/1986).

In conventional immunological assays, cardiac myosin of human origin is necessary as an antigen to obtain antibodies or as a standard to measure cardiac myosin concentrations. However, it has been quite difficult to obtain a large amount of cardiac myosin of human origin.

Under these circumstances, development of a method of steadily and abundantly producing human cardiac myosin has been urgently needed.

## SUMMARY OF THE INVENTION

We have made extensive research efforts regarding a method of producing human cardiac myosin by the use of recombinant DNA technology, which led to success in cloning a gene coding for human cardiac myosin as well as in revealing the DNA sequence of said gene. We have further found that recombinant human cardiac myosin can be steadily and abundantly produced by the use of the revealed gene coding for cardiac myosin and have thus arrived at the present invention.

More particularly, the present invention relates to a substantially pure recombinant human cardiac myosin and fragments thereof free from other proteins of human origin.

The present invention also relates to DNA sequences coding for the above-mentioned recombinant human cardiac myosin and for the fragments thereof.

Furthermore, the present invention relates to a method of producing the above-mentioned recombinant human cardiac myosin, to an expression vector to be used in this method and to a host cell transformed by this expression vector.

## BRIEF DESCRIPTION OF THE DRAWINGS

In the accompanying drawings:

FIG.1 is a chart showing restriction enzyme cleavage maps of the cDNA fragments with the insertions of plasmids pHMLC1A1 (upper) and pHMLC1B2 (lower).

The solid black portion indicates the open reading frame.

FIG. 2A is a coding map showing the DNA sequence of the cDNA coding for human atrial myosin light chain I and the amino acid sequence corresponding to the open reading frame of the cDNA.

FIG. 2B is a coding map showing the DNA sequence of the cDNA coding for human ventricular myosin light chain I and the amino acid sequence corresponding to the open reading frame of the cDNA.

FIG. 3 is a chart indicating the steps of construction of yeast expression vector YEp13-GAP-VLC.

FIG. 4 is a chart indicating the steps of construction of E. coli expression vector pDR-VLC4.

## DETAILED DESCRIPTION OF THE INVENTION

The following abbreviations are used herein:

Ala: L-Alanine
Arg: L-Arginine
Asn: L-Asparagine
Asp: L-Aspartic acid
Cys: L-Cysteine
Gln: L-Glutamine
Glu: L-Glutamic acid
Gly: Glycine
His: L-Histidine
Ile: L-Isoleucine
Leu: L-Leucine
Lys: L-Lysine
Met: L-Methionine
Phe: L-Phenylalanine
Pro: L-Proline
Ser: L-Serine
Thr: L-Threonine
Trp: L-Tryptophan
Tyr: L-Tyrosine
Val: L-Valine
A: 2'-Deoxyadenylic acid
C: 2'-Deoxycytidylic acid
T: Thymidylic acid
G: 2'-Deoxyguanylic acid
DNA: Deoxyribonucleic acid
RNA: Ribonucleic acid
mRNA: Messenger RNA
cDNA: Complementary DNA
Tris-HCl: Trisaminomethane-hydrochloric acid buffer solution
DTT: Dithiothreitol
ATP: Adenosine 5'-triphosphate
$MgCl_2$: Magnesium chloride
NaCl: Sodium chloride
EDTA: Ethylenediaminetetraacetic acid

KCl: Potassium chloride
unit: Unit of enzyme activity
dATP: 2'-Deoxyadenosine 5'-triphosphate
dCTP: 2'-Deoxycytidine 5'-triphosphate
dTTP: Thymidine 5'-triphosphate
dGTP: 2'-Deoxyguanosine 5'-triphosphate
dNTP: A mixture of dATP, dCTP, dGTP and dTTP
PMSF: Phenylmethanesulfonyl fluoride

The recombinant human cardiac myosin of the present invention, prepared by the use of recombinant DNA technology, is substantially pure human cardiac myosin free from other proteins of human origin. More specifically, an example of the recombinant human cardiac myosin has an amino acid sequence represented by the following formula (I).

Met-Ala-Pro-Lys-Lys-Pro-Glu-Pro-Lys-Lys-X1-
X2-Ala-Lys-X3-Ala-Pro-X4-X3-Ala-Pro-Ala-Pro-
Ala-Pro-Z ( I )

wherein, X1 is Glu or Asp, X2 is Ala or Asp, X3 is Pro or Ala, X4 is Ala or Lys, and Z is the amino acid sequence of either (II) or (III) shown below:

Pro Pro Glu Pro Glu Arg Pro Lys Glu Val Glu
Phe Asp Ala Ser Lys Ile Lys Ile Glu Phe Thr
Pro Glu Gln Ile Glu Glu Phe Lys Glu Ala Phe
Met Leu Phe Asp Arg Thr Pro Lys Cys Glu Met
Lys Ile Thr Tyr Gly Gln Cys Gly Asp Val Leu
Arg Ala Leu Gly Gln Asn Pro Thr Gln Ala Glu
Val Leu Arg Val Leu Gly Lys Pro Arg Gln Glu
Glu Leu Asn Thr Lys Met Met Asp Phe Glu Thr

Phe Leu Pro Met Leu Gln His Ile Ser Lys Asn
Lys Asp Thr Gly Thr Tyr Glu Asp Phe Val Glu
Gly Leu Arg Val Phe Asp Lys Glu Gly Asn Gly
Thr Val Met Gly Ala Glu Leu Arg His Val Leu
Ala Thr Leu Gly Glu Arg Leu Thr Glu Asp Glu
Val Glu Lys Leu Met Ala Gly Gln Glu Asp Ser
Asn Gly Cys Ile Asn Tyr Glu Ala Phe Val Lys
His Ile Met Ser Ser (II)

Ala Pro Ala Pro Ala Pro Glu Ala Pro Lys Glu
Pro Ala Phe Asp Pro Lys Ser Val Lys Ile Asp
Phe Thr Ala Glu Gln Ile Glu Glu Phe Lys Glu
Ala Phe Ser Leu Phe Asp Arg Thr Pro Thr Gly
Glu Met Lys Ile Thr Tyr Gly Gln Cys Gly Asp
Val Leu Arg Ala Leu Gly Gln Asn Pro Thr Asn
Ala Glu Val Leu Arg Val Leu Gly Lys Pro Lys
Pro Glu Glu Met Asn Val Lys Met Leu Asp Phe
Glu Thr Phe Leu Pro Ile Leu Gln His Ile Ser
Arg Asn Lys Glu Gln Gly Thr Glu Asp Asp Phe
Val Glu Gly Leu Arg Val Phe Glu Lys Glu Ser
Asn Gly Thr Asp Met Gly Ala Glu Leu Arg His
Val Leu Ala Thr Leu Gly Glu Lys Met Thr Glu
Val Glu Val Glu Gln Leu Leu Ala Gly Gln Glu
Asp Ala Asn Gly Cys Ile Asn Tyr Glu Ala Phe
Val Lys His Ile Met Ser Gly (III)

wherein, the cysteine residues may be present in the reduced form, or in the oxidized form in which intramolecular S-S bridge is formed between any two cysteine residues via a disulfide bond. Furthermore, one or more of amino acids may be substituted by other amino acids.

Examples of the recombinant human cardiac myosin having the amino acid sequence represented by the formula (I) shown above include the light chain I of recombinant human ventricular myosin having the amino acid sequence of the formula (IV) shown below and the light chain I of recombinant human atrial myosin having the amino acid sequence of the formula (V) shown below.

Met Ala Pro Lys Lys Pro Glu Pro Lys Lys Asp
Asp Ala Lys Ala Ala Pro Lys Ala Ala Pro Ala
Pro Ala Pro Pro Pro Glu Pro Glu Arg Pro Lys
Glu Val Glu Phe Asp Ala Ser Lys Ile Lys Ile
Glu Phe Thr Pro Glu Gln Ile Glu Glu Phe Lys
Glu Ala Phe Met Leu Phe Asp Arg Thr Pro Lys
Cys Glu Met Lys Ile Thr Tyr Gly Gln Cys Gly
Asp Val Leu Arg Ala Leu Gly Gln Asn Pro Thr
Gln Ala Glu Val Leu Arg Val Leu Gly Lys Pro

Arg Gln Glu Glu Leu Asn Thr Lys Met Met Asp
Phe Glu Thr Phe Leu Pro Met Leu Gln His Ile
Ser Lys Asn Lys Asp Thr Gly Thr Tyr Glu Asp
Phe Val Glu Gly Leu Arg Val Phe Asp Lys Glu
Gly Asn Gly Thr Val Met Gly Ala Glu Leu Arg
His Val Leu Ala Thr Leu Gly Glu Arg Leu Thr
Glu Asp Glu Val Glu Lys Leu Met Ala Gly Gln
Glu Asp Ser Asn Gly Cys Ile Asn Tyr Glu Ala
Phe Val Lys His Ile Met Ser Ser (IV)

Met Ala Pro Lys Lys Pro Glu Pro Lys Lys Glu
Ala Ala Lys Pro Ala Pro Ala Pro Ala Pro Ala
Pro Ala Pro Ala Pro Ala Pro Ala Pro Glu Ala
Pro Lys Glu Pro Ala Phe Asp Pro Lys Ser Val
Lys Ile Asp Phe Thr Ala Glu Gln Ile Glu Glu
Phe Lys Glu Ala Phe Ser Leu Phe Asp Arg Thr
Pro Thr Gly Glu Met Lys Ile Thr Tyr Gly Gln
Cys Gly Asp Val Leu Arg Ala Leu Gly Gln Asn
Pro Thr Asn Ala Glu Val Leu Arg Val Leu Gly
Lys Pro Lys Pro Glu Glu Met Asn Val Lys Met
Leu Asp Phe Glu Thr Phe Leu Pro Ile Leu Gln
His Ile Ser Arg Asn Lys Glu Gln Gly Thr Glu
Asp Asp Phe Val Glu Gly Leu Arg Val Phe Glu

Lys Glu Ser Asn Gly Thr Asp Met Gly Ala Glu
Leu Arg His Val Leu Ala Thr Leu Gly Glu Lys
Met Thr Glu Val Glu Val Glu Gln Leu Leu Ala
Gly Gln Glu Asp Ala Asn Gly Cys Ile Asn Tyr
Glu Ala Phe Val Lys His Ile Met Ser Gly

(V)

Furthermore, the recombinant human cardiac myosin of the present invention may be a polypeptide fragment having any part of the amino acid sequence of the formula (I) shown above. Examples of such polypeptides include a fragment in which one or more amino acids at the N-terminus and/or C-terminus are deleted, either consecutively or non-consecutively, and a fragment produced by treatment with various proteases such as serine protease, thiol protease, acid protease and metal protease. Among such fragments, those having poor homology to human skeletal myosin are especially useful as antigens to obtain antibodies more specific to human cardiac myosin.

The recombinant human cardiac myosin of the present invention can be prepared by recombinant DNA technology, more especifically, by following in order the steps of:

(a) isolating a cDNA clone coding for human cardiac myosin;

(b) introducing the cDNA obtained in the step (a) into an expression vector;

(c) transforming host cells by the expression vector obtained in the step (b);

(d) culturing the tranformants obtained in the step (c); and

(e) extracting the recombinant human cardiac myosin from the culture obtained in the culturing step (d) and purifying the same.

(a) Isolation of cDNA clone coding for human cardiac myosin:

An example of cDNA coding for human cardiac myosin has the DNA sequence of the following formula (XI):

Y4-Y9-Y7-Y14-Y14-Y7-Y16-Y7-Y14-Y14-W1-W2-Y9-
Y14-W3-Y9-Y7-W4-W3-Y9-Y7-Y9-Y7-Y9-Y7-X

(XI)

wherein Y4 is ATG coding for Met; Y7 is CCT, CCC, CCA or CCG coding for Pro; Y9 is GCT, GCC, GCA or GCG coding for Ala; Y14 is AAA or AAG coding for Lys; Y16 is GAA or GAG coding for Glu: W1 is GAA or GAG coding for Glu or GAT or GAC coding for Asp; W2 is GCT, GCC, GCA or GCG coding for Ala or GAT or GAC coding for Asp; W3 is CCT, CCC, CCA or CCG coding for Pro or GCT, GCC, GCA or GCG coding for Ala; W4 is GCT, GCC, GCA or GCG coding for Ala or AAA or AAG coding for Lys; and X is the DNA sequence of the formula (XII) or (XIII) shown below:

Y7-Y7-Y16-Y7-Y16-Y19-Y7-Y14-Y16-Y5-Y16-Y1-Y15-Y9-Y6-Y14-Y3-Y14-Y3-Y16-Y1-Y8-Y7-Y16-Y12-Y3-Y16-Y16-Y1-Y14-Y16-Y9-Y1-Y4-Y2-Y1-Y15-Y19-Y8-Y7-Y14-Y17-Y16-Y4-Y14-Y3-Y8-Y10-Y20-Y12-Y17-Y20-Y15-Y5-Y2-Y19-Y9-Y2-Y20-Y12-Y13-Y7-Y8-Y12-Y9-Y16-Y5-Y2-Y19-Y5-Y2-Y20-Y14-Y7-Y19-Y12-Y16-Y16-Y2-Y13-Y8-Y14-Y4-Y4-Y15-Y1-Y16-Y8-Y1-Y2-Y7-Y4-Y2-Y12-Y11-Y3-Y6-Y14-Y13-Y14-Y15-Y8-Y20-Y8-Y10-Y16-Y15-Y1-Y5-Y16-Y20-Y2-Y19-Y5-Y1-Y15-Y14-Y16-Y20-Y13-Y20-Y8-Y5-Y4-Y20-Y9-Y16-Y2-Y19-Y11-Y5-Y2-Y9-Y8-Y2-Y20-Y16-Y19-Y2-Y8-Y16-Y15-Y16-Y5-Y16-

Y14-Y2-Y4-Y9-Y20-Y12-Y16-Y15-Y6-Y13-Y20-Y17-
Y3-Y13-Y10-Y16-Y9-Y1-Y5-Y14-Y11-Y3-Y4-Y6-Y6-
Z (XII)

Y9-Y7-Y9-Y7-Y9-Y7-Y16-Y9-Y7-Y14-Y16-Y7-Y9-
Y1-Y15-Y7-Y14-Y6-Y5-Y14-Y3-Y15-Y1-Y8-Y9-Y16-
Y12-Y3-Y16-Y16-Y1-Y14-Y16-Y9-Y1-Y6-Y2-Y1-
Y15-Y19-Y8-Y7-Y8-Y20-Y16-Y4-Y14-Y3-Y8-Y10-
Y20-Y12-Y17-Y20-Y15-Y5-Y2-Y19-Y9-Y2-Y20-Y12-
Y13-Y7-Y8-Y13-Y9-Y16-Y5-Y2-Y19-Y5-Y2-Y20-
Y14-Y7-Y14-Y7-Y16-Y16-Y4-Y13-Y5-Y14-Y4-Y2-
Y15-Y1-Y16-Y8-Y1-Y2-Y7-Y3-Y2-Y12-Y11-Y3-Y6-
Y19-Y13-Y14-Y16-Y12-Y20-Y8-Y16-Y15-Y15-Y1-
Y5-Y16-Y20-Y2-Y19-Y5-Y1-Y16-Y14-Y16-Y6-Y13-
Y20-Y8-Y15-Y4-Y20-Y9-Y16-Y2-Y19-Y11-Y5-Y2-
Y9-Y8-Y2-Y20-Y16-Y14-Y4-Y8-Y16-Y5-Y16-Y5-
Y16-Y12-Y2-Y2-Y9-Y20-Y12-Y16-Y15-Y9-Y13-Y20-
Y17-Y3-Y13-Y10-Y16-Y9-Y1-Y5-Y14-Y11-Y3-Y4-
6-Y20-Z (XI)

wherein Y1 is TTT or TTC coding for Phe; Y2 is TTA, TTG, CTT, CTC, CTA or CTG coding for Leu; Y3 is ATT, ATC or ATA coding for Ile; Y5 is GTT, GTC, GTA or GTG coding for Val; Y6 is TCT, TCC, TCA, TCG, AGT or AGC coding for Ser; Y8 is ACT, ACC, ACA or ACG coding for Thr; Y10 is TAT or TAC coding for Tyr; Y11 is CAT or CAC coding for His; Y12 is CAA or CAG coding for Gln; Y13 is AAT or AAC coding for Asn; Y15 is GAT or GAC coding for Asp; Y17 is TGT or TGC coding for Cys; Y18 is TGG coding for Trp; Y19 is CGT, CGC, CGA, CGG, AGA or AGG coding for Arg; Y20 is GGT, GGC, GGA or GGG coding for Gly; and Z is a stop codon which is TAA, TAG or TGA.

In the case where expression is directed to a polypeptide fragment of human cardiac myosin, only the DNA sequence, corresponding to that fragment, of the cDNA set forth above is used.

Examples of the cDNA of the formula (XI) shown above include the cDNA, coding for the light chain I of human ventricular myosin, of the formula (XIV) shown below and the cDNA, coding for the light chain I of the human atrial myosin, of the formula (XV) shown below:

ATG GCC CCC AAA AAG CCA GAG CCC AAG AAG GAT
GAT GCC AAG GCA GCC CCC AAG GCA GCT CCA GCT
CCC GCA CCT CCC CCT GAG CCT GAG CGC CCT AAG
GAG GTC GAG TTT GAT GCT TCC AAG ATC AAG ATT
GAG TTC ACA CCT GAG CAG ATT GAA GAG TTC AAG
GAA GCC TTC ATG CTG TTC GAC CGC ACA CCC AAG
TGT GAG ATG AAG ATC ACC TAC GGG CAG TGT GGG
GAT GTC CTG CGG GCG CTG GGC CAG AAC CCC ACA
CAG GCA GAA GTG CTC CGT GTC CTG GGG AAG CCA
AGA CAG GAA GAG CTC AAT ACC AAG ATG ATG GAC
TTT GAA ACT TTC CTG CCT ATG CTC CAG CAC ATT

TCC AAG AAC AAG GAC ACA GGC ACC TAT GAG GAC
TTC GTG GAG GGG CTG CGG GTC TTC GAC AAG GAG
GGC AAT GGC ACT GTC ATG GGT GCT GAG CTT CGC
CAC GTG CTG GCC ACG CTG GGT GAG AGG CTG ACA
GAA GAC GAA GTG GAG AAG TTG ATG GCT GGG CAA
GAG GAC TCC AAT GGC TGC ATC AAC TAT GAA GCA
TTT GTG AAG CAC ATC ATG TCC AGC Z (XV)

ATG GCT CCC AAG AAG CCT GAG CCT AAG AAG GAG
GCA GCC AAG CCA GCT CCA GCT CCA GCT CCA GCC
CCT GCA CCA GCC CCT GCC CCA GCT CCT GAG GCT
CCC AAG GAA CCT GCC TTT GAC CCC AAG AGT GTA
AAG ATA GAC TTC ACT GCC GAG CAG ATT GAA GAG
TTA AAA GAG GCC TTT TCA TTG TTT GAC CGG ACC
CCG ACT GGA GAG ATG AAG ATC ACC TAC GGC CAG
TGC GGG GAT GTA CTG CGG GCC CTG GGC CAG AAC
CCT ACC AAT GCC GAG GTG CTG CGT GTG CTG GGC
AAG CCC AAG CCT GAA GAG ATG AAT GTC AAG ATG
CTG GAC TTT GAG ACG TTC TTG CCC ATC CTG CAG
CAC ATT TCC CGC AAC AAG GAG CAG GGC ACC TAT
GAG GAC TTC GTG GAG GGC CTG CGT GTC TTT GAG
AAG GAG AGC AAT GGC ACG GAC ATG GGT GCT GAG

CTT CGG CAC GTC CTT GCC ACC CTG GGA GAG AAG
ATG ACT GAG GCT GAA GTG GAG CAG CTG TTG GCT
GGG CAA GAG GAT GCC AAT GGC TGC ATC AAT TAT
GAA GCC TTT GTC AAG CAC ATC ATG TCA GGG Z

(XV)

wherein Z is a stop codon which is TAA, TAG or TGA.

These genes coding for human cardiac myosin can be prepared by an ordinary method such as preparation from cDNA, or chemical synthesis by the use of the phosphotriester method or the phosphite triester method.

For example, the preparation from cDNA can be accomplished by following the procedure comprising the steps of 1) preparing a cDNA library, 2) screening a cDNA fragment coding for a desired human cardiac myosin and 3) preparing an open reading frame (ORF) DNA fragment.

1) Preparation of cDNA library

The cDNA library can be prepared by an ordinary method. For example, the whole RNA fraction is extracted from the postmortem human heart by an ordinary method and passed through an oligo (dT) cellulose column to obtain poly(A)-RNA. With the use of the resultant mRNA (poly(A)-RNA) as template, double strand cDNA is synthesized according to an ordinary method, such as the Gubler-Hoffman method (Gubler, U. and Hoffman, J., Gene, 25, 263 (1983)) or the Okayama-Berg method (Okayama, H. and Berg. P., Mol. Cell Biol., 2, 161 (1982)). Preparation of the cDNA library from the cDNA thus synthesized can be carried out by an ordinary method, such as the in vitro packaging method with the use of a phage vector such as lambda-gt11 (Young, R. A. and Davis, R. W., Proc. Natl. Acad. Sci. USA, 80, 1194 (1983)) or the Okayama-Berg method with the use of a plasmid vector (loc cit).

2) Screening of cDNA library and determination of cDNA nucleotide sequence

The screening of a cDNA clone coding for cardiac myosin from the cDNA library can be carried out by the plaque hybridization method with the use of a phage vector (Maniatis et al., Molecular cloning, Cold Spring Harbor Laboratory, Cold Spring Harbor, New York, 1982) or by the colony hybridization method with the use of a plasmid vector (Perbal, B., A Practical Guide to Molecular Cloning, John Wiley & Sons, New York (1984)). Examples of probes to be used in the screening include a cloned cDNA among myosin of xenogenic animals which is highly homologous, in respect to the amino acid sequence, to the human cardiac myosin to be screened. For example, cDNA of the light chain of chicken skeletal myosin (Nucleic Acids Res., 10, 6099 (1982)) or a synthesized oligonucleotide corresponding to a part of the previously known amino acid sequence of the light chain of human cardiac myosin (Klotz, C. et al., Circ. Res., 50, 201 (1982)) can be used. By the use of the probe, a clone which hybridizes with the probe is selected, and the recombinant phage DNA (or the recombinant plasmid DNA) thereof is prepared by an ordinary method. The DNA sequence of the inserted cDNA fragment is determined by the dideoxy chain terminator method (Sanger, F. et al., Proc. Natl. Acad. Sci. USA, 74, 5463 (1977)) or by the Maxam-Gilbert method (Methods in Enzymology, 65, 497 (1980)).

The amino acid sequence of the protein encoded by the DNA, which was predicted from the determined DNA sequence, the molecular weight and the amino acid composition of the resultant protein were compared with those of human cardiac myosin and, if necessary, further with the partial amino acid sequence of the light chain of human cardiac myosin previously reported by Klotz et al. (Circ. Res., 50, 201 (1982)) so as to obtain the cDNA fragment coding for the objective human cardiac myosin.

3) Preparation of human cardiac myosin ORF DNA

In the case where the cDNA fragment coding for human cardiac myosin, obtained as described above, includes the 5′- and 3′-untranslated regions derived from the mRNA, the resultant cDNA is preferably prepared so as to possibly exclude such regions. Such nonessential regions can be eliminated by an ordinary treatment with enzymes, for example, restriction endonucleases and exonucleases such as nuclease Bal31.

(b) Introduction of cDNA obtained in the step (a) into a vector:

The cDNA obtained in the step (a) above is inserted into the region downstream of an expression promoter in the coordinate direction for transcription so as to construct an expression vector.

Usable expression vectors may vary depending on the host species. In the case where E. coli is used as a host, vectors containing a promoter such as the tryptophan operon promoter (trp promoter), the lactose operon promoter (lac promoter), the tac promoter or the $P_L$ promoter can be used. Examples of such vectors include pKK223-3, pDR540, pDR720, pYEJ001 and pRL-lambda (all being products of Pharmacia).

Furthermore, in the case where yeast is used as a host, vectors containing a promoter of the gene coding for enzymes can be used. Examples of such enzymes include those glycolytic enzymes, such as enolase, glyceraldehyde-3-phosphate dehydrogenase (hereinafter referred to as "GAP-DH"), hexokinase, pyruvate decarboxylase, phosphofructokinase, glucose-6-phosphate isomerase, 3-phosphoglycerate mutase, pyruvate kinase, triphosphate isomerase, phosphoglucose isomerase and glucokinase, and 3-phosphoglycerate kinase, alcohol dehydrogenase, alkaline phosphatase and repressive acid phosphatase. Particularly suitable vectors are those having high promoter activity. Examples of such vectors are those carrying promoters for GAP-DH (J. Biol. Chem., 254, 9839 (1979)), 3-phosphoglycerate kinase (J. Biol. Chem., 255, 2073 (1980)) and suppressive acid phosphatase (Nucleic Acids Res., 11, 1657 (1983)).

The construction of the expression vector may be carried out according to an ordinary method. For example, in the case where pDR540 (Boer, H. et al., Proc. Natl. Acad. Sci. USA, 80, 21 (1983)) is used, a DNA fragment having a gene to be expressed is made by digesting with BamHI or with a restriction enzyme which produces the same structure as the cohesive end produced by the digestion with BamHI, since the cloning site of pDR540 in the downstream region of the promoter is the BamHI site. This DNA fragment is then ligated into the downstream region of the SD (Shine-Dalgarno) sequence located in the region downstream of the promoter so as to construct an expression vector. Furthermore, the distance from the SD sequence to the translational start codon (ATG) is preferably about 7 to 12 base pairs (bp). The DNA fragment coding for the objective protein is preferably prepared so as to maintain such distance. The cDNA to be inserted into such a vector can be prepared by an ordinary method. For example, after the DNA fragment is cleaved at the 5′-terminus and the 3′-terminus with suitable restriction enzymes, an unnecessary part is removed from the cleaved sites using nuclease Bal31 or nuclease lambda exoIII, and then the blunt ends are made using the Klenow fragment. Subsequently, this DNA fragment is ligated at the blunt ends to the BamHI linker using T4 DNA ligase and then digested with BamHI so as to be cloned in a vector such as M13mp18 (Messing, J., Methods in Enzymology, 101, 20 (1983)). The DNA sequences of the terminal ends of the cloned DNA fragments are analyzed, for example, by the dideoxy chain terminator method so as to select objective clones.

(c) Transformation of a host cell by the expression vector obtained in the step (b):

Transformation of a host cell by the expression vector obtained in the step (b) above can be carried out by an ordinary method. For example, in the case where E. coli-plasmid vector is used, the calcium chloride method (Mandel, M and A. Higa, J. Mol. Biol., 53, 154 (1970)) is applicable, or in the case where yeast is used as a host, the protoplast method (Hinnen, A. et al., Proc. Natl. Acad. Sci. USA, 75, 1929 (1978)) or the lithium acetate method (Ito, H. et al., J. Bacteriol., 153, 163 (1983)) is applicable.

(d) Cultivation of transformants obtained in the step (c):

The transformants obtained in the step (c) above may be cultivated using a medium containing nutrient sources such as carbon sources or nitrogen sources according to an ordinary method.

Examples of the carbon sources include carbohydrates such as glucose, sucrose, fructose or dextrin, and examples of the nitrogen sources include peptone, meat extract, soy bean meal, yeast extract, various amino acids and ammonium chloride. In addition to the carbon sources and nitrogen sources, inorganic salts, such as sodium chloride, potassium chloride, magnesium sulfate and sodium hydrogen phosphate, growth-promoting factors, such as thiamin, riboflavin, biotin, pantothenic acid and vitamin $B_2$, and antibiotics, such as ampicillin and tetracycline, may be optionally added to the medium.

As to the cultivation of the transformant, more specifically, for example, in the case where E. coli JM105 (Messing, J., Methods in Enzymology, 101, 20 (1980))-plasmid vector pDR540 is used, cultivation can be carried out in 2 x YT medium (Messing, J., Methods in Enzymology, 100, 20 (1983)), LB medium (Maniatis, T. et al., Molecular Cloning, Cold Spring Harbor Laboratory, Cold Spring Harbor, New York (1982)) or the like, which allows the growth of E. coli JM105, with an addition of an appropriate amount of ampicillin so as to prevent deletion of the plasmids, at 20 to 40° C, if necessary with aeration and stirring.

Further, in the case where induction of expression is needed during the incubation, the induction of the expression may be carried out according to the method ordinarily applied to the promoter used. For example, in order to induce the transcription of the expression promoter tac of pDR540, isopropyl-beta-D-thiogalactopyranoside (IPTG) is added when the transformants are grown to some extent, and then cultivation is further continued for several hours.

In the case where yeast is used as a host, in the same manner as described for E. coli above, the cultivation can be carried out in a medium containing the above-mentioned medium constituents which allow the growth of the transformants, more specifically, in the medium containing 2 % glucose and 0.7 % Yeast Nitrogen Base supplemented with nutrient sources according to requirement of the transformants, at 20 to 40° C, if necessary with aeration and stirring. Also in the case where yeast is used as a host and induction of expression of a promoter for expression is needed, the expression induction can be carried out according to the method ordinarily applied to the promoter used.

(e) Extraction and purification of recombinant human cardiac myosin from the culture:

The extraction of recombinant human cardiac myosin can be carried out substantially in an ordinary method. For example, the cultured cells are recovered by centrifugation and suspended in an appropriate buffer. An appropriate protease inhibitor, such as EDTA and PMSF, is preferably added to the buffer and all the procedures are preferably carried out at a low temperature, so that degradation by proteases or the like in the host cells is prevented as much as possible. The method for extracting recombinant human cardiac myosin from the cultured cells may be optionally selected from those appropriate to the individual host cells. For example, if E. coli is used as a host, ultrasonication, treatment with an enzyme, such as lysozyme, or osmotic shock can be applied. Furthermore, if yeast is used as a host, for example, grinding with glass beads or zymolyase treatment can be applied. The extract is centrifuged to remove cell debris, and then the resultant supernatant is purified, for example, by ion exchange chromatography and gel filtration (Experimental Methods in Biochemistry; Muscle, Vol. 15, p.3-12, Tokyo Kagaku Dojin Co., Ltd. (1975)), preparative gel electrophoresis (Circ. Res., 36, 208 (1984)), or affinity chromatography using a monoclonal antibody having high specificity against human cardiac myosin (Japanese Patent Laid-Open Pub. No. 286752/1986) so as to obtain recombinant human cardiac myosin.

The recombinant human cardiac myosin thus obtained is useful as a standard in the immunological measurement of human cardiac myosin or as an antigen to obtain an antibody having higher specificity to and affinity for cardiac myosin.

For example, in the case where the recombinant human cardiac myosin is used as a standard in the immunological measurement, the above-mentioned recombinant human cardiac myosin of the present invention is prepared in a lyophilized form or in solution of a desired concentration, if necessary, with an addition of a stabilizer (e.g., animal serum, saccharides such as mannitol and sucrose, polyhydric alcohols such as glycerin) and dispensed into appropriate containers such as vials so as to affix to a measuring kit.

Furthermore, in the case where the recombinant human cardiac myosin is used as an antigen for antibody preparation, the recombinant human cardiac myosin or fragments thereof of the present invention can be mixed with complete Freund's adjuvant or aluminum hydroxide. The resultant admixture is used as an antigen for antibody preparation.

According to the present invention, it is possible to steadily provide human cardiac myosin homogeneous in quality, which has been otherwise difficult to obtain or prepare. The recombinant human cardiac myosin produced by recombinant DNA technology is useful not only as a standard for immunological measurement but also as an antigen to obtain antibody having higher specificity and affinity.

## EXAMPLE

The following example serves to illustrate the present invention more specifically.

### (1) Isolation of cDNA clone coding for human cardiac myosin light chain I:

#### (1-a) Construction of cDNA library

RNA was extracted from the heart resected from a fetus, who had died 17 weeks after gestation, and stored at -70°C, according to the lithium chloride method (Auffray, C. and Rougeon, F., Eur. J. Biochem., 107, 303 (1980)). The extracted RNA was applied to oligo (dT) cellulose column chromatography according to the method of Maniatis et al. (Molecular Cloning, Cold Spring Harbor Laboratory, Cold Spring Harbor, New York (1982)) to obtain purified poly(A) RNA. By using the purified poly(A) RNA, a double-stranded cDNA was prepared according to the Gubler-Hoffman method (Gene, 25, 263 (1983)). The first strand was constructed using the oligo (dT) 12-18 (Pharmacia) as a primer by using avian myeloblastosis virus reverse transcriptase (Boehringer Mannheim). The second strand was synthesized by using RNase H (Takara Shuzo Co., Ltd.) and DNA polymerase I (Takara Shuzo Co., Ltd.). The resultant double-stranded cDNA (2 micrograms) was incubated in 50 microliters of a reaction mixture [100 mM Tris-HCl (pH 7.5), 2 mM DTT, 10 mM EDTA, 80 micromols/liter S-adenosyl-L-methionine, and 10 units of EcoRI methylase (Takara Shuzo Co., Ltd.)] at 37°C for 1 hour, and the mixture was then precipitated with ethanol to recover DNA. The recovered DNA was allowed to react in 50 microliters of a reaction mixture [67 mM potassium phosphate buffer (pH 7.4), 6.7 mM $MgCl_2$, 1 mM 2-mercaptoethanol, 33 micromols/liter dNTP, and 2 units of Klenow fragment (Takara Shuzo Co., Ltd.)] at 37°C for 1 hour to obtain a blunt-ended cDNA fragment. The blunt-ended cDNA fragment was incubated in 50 microliters of a reaction mixture [66 mM Tris-HCl (pH 7.6), 6.6 mM $MgCl_2$, 10 mM DTT, 0.1 mM ATP, 100 units of T4 DNA ligase (Takara Shuzo Co., Ltd.), and 10 ng/microliter pEcoRI linker (Takara Shuzo Co., Ltd.)] at 12°C for 12 hours, and the mixture was precipitated with ethanol to recover DNA. The DNA was then incubated in 50 microliters of a reaction mixture [100 mM Tris-HCl (pH 7.5), 7 mM $MgCl_2$, 50 mM NaCl, 7 mM 2-mercaptoethanol, 0.01 % bovine serum albumin, and 20 units of EcoRI (Takara Shuzo Co., Ltd.)] at 37°C for 2 hours to obtain a DNA fragment having EcoRI ends.

An excess of pEcoRI linker remaining in the reaction mixture was removed by Sephadex G-100 (Pharmacia) column chromatography.

One microgram of DNA of lambda-gt11 (Young, R. A. and Davis, R. W., Proc. Natl. Acad. Sci. USA, 80, 1194 (1983); Promega-Bioteck) was incubated in 20 microliters of a reaction mixture [100 mM Tris-HCl (pH 7.5), 7 mM $MgCl_2$, 50 mM NaCl, 7 mM 2-mercaptoethanol, 0.01 % bovine serum albumin, and 20 units of EcoRI] at 37°C for 2 hours, and the mixture was precipitated with ethanol to recover DNA. The DNA was then incubated in 20 microliters of a reaction mixture [1 M Tris-HCl (pH 8.0), and 1 unit of E. coli alkaline phosphatase (Takara Shuzo Co., Ltd.)] at 37°C for 2 hours, and the reaction mixture was subjected to phenol-chloroform extraction and ethanol precipitation to recover the DNA.

The EcoRI-ended cDNA fragment was admixed with the lambda-gt11 DNA treated both with EcoRI and alkaline phosphatase, and the mixture was allowed to react in 50 microliters of a reaction mixture [66 mM Tris-HCl (pH 7.6), 6.6 mM $MgCl_2$, 10 mM DTT, 0.1 mM ATP, and 100 units of T4 DNA ligase] at 16°C for 12 hours. The reaction mixture was subjected to in vitro packaging by using a lambda DNA in vitro packaging kit (Amersham) according to the method of Williams et al. (Genetic Engineering, 2, Plenum Press (1980)) and then E. coli Y1088 (ATCC No. 37195) was transduced, whereby the cDNA library of 5 x $10^5$ or more was constructed.

#### (1-b) Screening of the cDNA library and determination of DNA sequence of the cDNA

For the selection of human cardiac myosin light chain cDNA from the cDNA library, the screening was carried out according to the plaque hybridization method (Molecular Cloning, loc cit) using, as a probe, plasmid pSMA1-1 (Nabeshima, Y. et al., Nucleic Acids Res., 10, 6099 (1982)) with an insertion of chicken skeletal myosin light chain I cDNA. As a result, of 2 x $10^5$ plaques, 25 plaques were hybridized with the probe. Recombinant phage DNA was prepared from the clones hybridized with the probe according to the plate lysate method (Molecular Cloning, loc cit). The inserted EcoRI fragment was subcloned into plasmid vector pUC13 (Vieiru, J. and Messing, J., Gene, 19, 259 (1982)) and subjected to structure analysis by cleavage with various restriction endonucleases (FIG. 1). Of the analyzed clones, the DNA sequences of the two clones exhibiting different restriction endonuclease cleavage patterns, i.e., the cDNA fragments with an insertion of pHMLC1A1 and pHMLC1B2, were determined according to the dideoxy chain terminator method (Sanger, F. et al., Proc. Natl. Acad. Sci. USA, 74, 5463 (1977)) (FIG. 2). From the DNA sequences thus obtained, the amino acid sequences of the proteins were predicted, and the amino acid compositions were compared with those for the purified human cardiac myosin light chains reported by Vincent et al. (Eur. J. Biochem., 148, 135 (1985)). The amino acid compositions of the proteins from pHMLC1A1 and pHMLC1B2 were identical to those for human atrial myosin and human ventricular myosin, respectively. Furthermore, as to pHMLC1B2, its amino acid sequence was identical to the partial amino acid sequence of the human ventricular myosin light chain I, reported by Klotz et al. (Circ. Res., 50, 201 (1982)), except that Asn at the position 140 and Ser at the position 190 were replaced by Asp and Gly, respectively. Furthermore, by the use of the RNA blot hybridization method (Molecular Cloning, loc cit), in which pHMLC1A1 (3′ HinfI fragment) and pHMLC1B2 (3′ BglI-EcoRI fragment), as probes, and RNA extracted from human ventricles and atria were used, it was confirmed that the cDNA's of human atrial myosin light chain I and human ventricular myosin light chain I were cloned into pHMC1A1 and pHMLC1B2, respectively.

Further, E. coli JM105 transformed with pHMLC1AI [E. coli TNC111 (JM105/pHMLC1A1)] and with pHMLC1B2 [E. coli TNC112 (JM105/pHMLC1B2)} were deposited at the Fermentation Research Institute, Agency of Industrial Science and Technology, Japan, under the deposit Nos. 9887 and 9888, respectively, E. coli TNC111 and E. coli TNC112 now bearing the accession numbers FERM BP-2253 and FERM BP-2254 under the Budapest Treaty on the International Recognition of the Deposit of Microorganism for the Purposes of Patent Procedure.

(2) Production of human ventricular myosin light chain I in yeast:

(2-a) Preparation of open reading frame (ORF) of human ventricular myosin light chain I

Two micrograms of pHMLC1B2 DNA was allowed to react in 50 microliters of a reaction mixture [10 mM Tris-HCl (pH 7.5), 10 mM $MgCl_2$, 50 mM KCl, 1 mM DTT, 10 units of AatII (New England Biolabs), and 10 units of RsaI (Takara Shuzo Co., Ltd.)] at 37°C for 5 hours and DNA was precipitated with ethanol. The DNA was then allowed to react in 50 microliters of a reaction mixture [33 mM Tris-aminomethane acetate (pH 7.9), 66 mM potassium acetate, 10 mM magnesium acetate, 0.5 mM DTT, 0.01 % bovine serum albumin 0.1 mM dNTP, and 10 units of T4 DNA polymerase] at 37°C for 5 minutes. After the reaction, DNA was recovered by ethanol precipitation, The DNA was allowed to react in 50 microliters of a reaction mixture [66 mM Tris-HCl (pH 7.6), 6.6 mM $MgCl_2$, 10 mM DTT, 0.1 mM ATP, 0.1 ng/microliter, pBamHI linker (5′p-CGGATTCCG-3′ (Takara Shuzo Co., Ltd.), and 100 units of T4 DNA ligase] at 12°C for 15 hours to ligate BamHI linker. The resultant DNA was again recovered by ethanol precipitation, allowed to react in 50 microliters of a reaction mixture [10 mM Tris-HCl (pH 7.5), 10 mM $MgCl_2$, 100 mM NaCl, 6 mM 2-mercaptoethanol, and 20 units of BamHI] at 37°C for 5 hours, and then recovered by ethanol precipitation. This DNA was mixed with 0.5 microgram of M13mp18 (Gene, 33, 103 (1985); Takara Shuzo Co., Ltd.) DNA, which had been allowed to react in 20 microliters of a reaction mixture [10 mM Tris-HCl (pH 7.5), 10 mM $MgCl_2$, 100 mM NaCl, 6 mM 2-mercaptoethanol, and 20 units of BamHI] at 37°C for 3 hours, and the mixture was allowed to react in 50 microliters of a reaction mixture [66 mM Tris-HCl (pH 7.6), 6.6 mM $MgCl_2$, 10 mM DTT, 0.1 mM ATP, and 100 units of T4 DNA ligase] at 16°C for 5 hours. Ten microliters of the reaction mixture was used for transformation of E. coli JM105 according to the method of Messing et al. (Methods in Enzymology, 101, 20 (1983)). From the plaques obtained, M13mp18-VCL was selected. The M13mp18-VLC is a fragment containing the RsaI-AatII fragment having BamHI ends at both termini which includes the open reading frame for human ventricular myosin light chain I.

(2-b) Construction of expression vector YEp13-GAP-VLC

Two micrograms of M13mp18-VLC DNA and 0.5 microgram of YEp13-GAPSH (Japanese Patent Application No. 272697/1986) DNA were allowed to react independently in 20 microliters of a reaction mixture [10 mM Tris-HCl (pH 7.5), 10 mM $MgCl_2$, 100 mM NaCl, 6 mM 2-mercaptoethanol, and 20 units of BamHI] at 37° C for 5 hours and precipitated with ethanol. The two DNA's were then allowed to react in 50 microliters of a reaction mixture [66 mM Tris-HCl (pH 7.6), 6.6 Mm $MgCl_2$, 10 mM DTT, 0.1 mM ATP, and 100 units of T4 DNA ligase] at 16° C for 12 hours. Twenty microliters of this reaction mixture was used for transformation of E. coli JM105 by the calcium chloride method, and then YEp13-GAP-VLC was selected from the resultant ampicillin-resistant transformants. The YEp-13-GAP-VLC is a plasmid having, in the coordinate direction for transcription, an insertion of the BamHI fragment containing the open reading frame for human ventricular myosin light chain I at the BamHI cleavage site downstream of yeast glyceraldehyde-3-phosphate dehydrogenase promoter.

(2-c) Expression of human ventricular myosin light chain in yeast carrying YEp13-GAP-VLC

With the plasmid YEp13-GAP-VLC DNA, yeast AH22 (a, leu2-3, leu2-112, his4-519, can-1; ATCC No. 38626) was transformed according to the lithium acetate method (J. Bacteriol., 153, 163 (1983)). The resultant transformants were cultivated in 10 ml of medium [2% glucose, 0.7% yeast nitrogen base (Difco Laboratories), and 20 micrograms/ml histidine] at 30° C for 15 hours. Cells were recovered at 8,000 x g for 10 minutes by centrifugation, suspended in 100 microliters of buffer [50 mM Tris-HCl (pH 7.8), 20 mM EDTA, 1 mM PMSF, and 50 micromols/liters 2-mercaptoethanol] and ground by means of the vortex treatment with an addition of 100 mg of glass beads. The suspension was centrifuged at 1,000 x g for 10 minutes, and the resultant supernatant was analyzed as a sample for the human cardiac myosin light chain according to the method of Yazaki et al. (Japanese Patent Laid-Open Pub. No. 286752/1986). Further, the protein concentration of the sample was determined by using a protein assay kit (Bio-Rad Laboratories). As a result, production of 260 ng myosin/mg protein in the yeast was confirmed.

(3) Production of human ventricular myosin light chain I in E. coli:

(3-a) Preparation of ORF DNA of human ventricular myosin light chain I

(3-a-1) Preparation of 5′-ORF DNA of human ventricular myosin light chain I

One microgram of pHMLC1B2 DNA was allowed to react in 50 microliters of a reaction mixture [100 mM Tris-HCl (pH 7.5), 7 mM $MgCl_2$, 50 mM NaCl, 7 mM 2-mercaptoethanol, 0.01 % bovine serum albumin, 20 units of EcoRI (Takara Shuzo Co., Ltd.), and 20 units of SacI (Takara Shuzo Co., Ltd.)] at 37° C for 2 hours. Then the resultant reaction mixture was mixed with 0.5 microgram of pHSG398 (Takara Shuzo Co., Ltd.), which had been allowed to react in 20 microliters of a reaction mixtures [100 mM Tris-HCl (pH 7.5), 7 mM $MgCl_2$, 50 mM NaCl, 7 mM 2-mercaptoethanol, 0.01 % bovine serum albumin, and 10 units each of EcoRI and SacI] at 37° C for 2 hours. The resultant mixture was allowed to react in 50 microliters of a reaction mixture [66 mM Tris-HCl (pH 7.6), 6.6 mM $MgCl_2$, 10 mM DTT, 0.1 mM ATP, and 100 units of T4 DNA ligase] at 16° C for 2 hours. By using 20 microliters of the resultant reaction mixture, E. coli JM105 (Gene, 19, 269 (1982)) was transformed according to the calcium chloride method (J. Mol. Biol., 53, 159 (1970)). From chloramphenicol-resistant, beta-galactosidase alpha-donor non-producing tranformants thus obtained, pHSG-5′-VLC was selected. The pHSG-5′-VLC is a clone in which the 5′-region of the open reading frame of human ventricular myosin light chain I of pHMLC1B2 is cloned at the EcoRI and SacI cleavage sites of pHSG398. Fifteen micrograms of pHSG-5′-VLC DNA was allowed to react in 200 microliters of a reaction mixture [100 mM Tris-HCl (pH 7.5), 7 mM $MgCl_2$, 50 mM NaCl, 6 mM 2-mercaptoethanol, and 30 units of EcoRI] at 37° C for 5 hours. pHSG-5′-VLC DNA digested with EcoRI was allowed to react with 0.08 unit of Bal31 nuclease (Takara Shuzo Co., Ltd.) in 200 microliters of a reaction mixture [20 mM Tris-HCl (pH 8.0) 12 mM $CaCl_2$, 12 mM $MgCl_2$, 0.2 M NaCl, and 2 mM EDTA] at 30° C for 8 minutes for partial digestion from the EcoRI cleavage sites. After the reaction, with an addition of 20 microliters of 0.2 M ethyleneglycol bis(2-aminoethylether)tetraacetic acid (EGTA), DNA was recovered by ethanol precipitation. Furthermore, for blunting of the termini, the recovered DNA was allowed to react in 100 microliters of a reaction mixture [33 mM trisaminomethane-acetate buffer (pH 7.9), 66 mM potassium acetate, 10 mM magnesium acetate, 0.5 mM DTT, 0.01% bovine serum albumin, 0.1 mM dNTP, and 10 units of T4 DNA polymerase] at 37° C for 5 minutes. After ethanol precipitation, with an addition of 5 micrograms of pBamHI linker (5′p-GAGGATCCTC-3′; Yamasa Shoyu, K. K., Japan), the DNA recovered by ethanol precipitation was allowed to react in 50 microliters of a reaction mixture [66 mM Tris-HCl (pH 7.6), 6.6 mM $MgCl_2$, 10 mM DTT, 1 mM ATP, and 100 units of T4 DNA ligase] at 12°C for 4 hours. After precipitation with ethanol, the DNA recovered was allowed to react in 50 microliters of a reaction mixture [10 mM Tris-HCl (pH 7.5), 10 mM $MgCL_2$, 100 mM NaCl, 6 mM 2-mercaptoethanol, and 20 units each of BamHI and SacI] at 37°C for 5 hours to obtain a BamHI-SacI DNA fragment.

In order to clone the BamHI-SacI DNA fragment containing the 5′-region of human ventricular myosin light chain I gene and determine the DNA sequence of the clone, one microgram of pUC18 (Gene, 33, (1985); available from Takara Shuzo Co., Ltd.) DNA was allowed to react in 50 microliters of a reaction mixture [10 mM tris-HCl (pH 7.5), 10 Mm $MgCl_2$, 100 mM NaCl, 6 mM 2-mercaptoethanol, and 10 units each of BamHI and SacI] at 37°C for 5 hours. After ethanol precipitation, the resultant fragment was mixed with pHMLC1B2 DNA, which had been digested with BamHI and SacI after Bal31 treatment as described above, and allowed to react in 50 microliters of a reaction mixture [66 mM Tris-HCl (pH 7.6), 6.6 mM $MgCl_2$, 10 mM DTT, 1 mM ATP, and 100 units of T4 DNA ligase] at 16° for 12 hours. By using 20 microliters of this reaction mixture, E. coli JM105 was transformed according to the calcium chloride method, and selection was carried out for ampicillin-resistant, beta-galactosidase alpha-donor non-producing transformants. From the selected transformants, a plasmid DNA was prepared by the alkaline method (Nucleic Acids Res., 7, 1513 (1979)) and analyzed by digestion with various restriction enzymes associated with polyacrylamide gel electrophoresis and by DNA sequence determination by the dideoxy chain terminator method so as to select pUC-5′-VLC4. The pUC-5′-VLC4 is a fragment containing the BamHI-SacI DNA fragment having an insertion of the BamHI linker 7 bp upstream to the 5′-terminus from the translation start codon of human ventricular myosin light chain I.

(3-a-2) Preparation of 3′-ORF DNA of human ventricular myosin light chain I

In order to prepare 3′-region of human ventricular myosin light chain I gene, 2 micrograms of pHMLC1B2 DNA was incubated in 50 microliters of a reaction mixture [10 mM Tris-HCl (pH 7.5), 10 mM $MgCl_2$, 50 mM KCl, 1 mM DTT, and 10 units of AatII (New England Biolabs)] at 37°C for 5 hours. After ethanol precipitation, the DNA recovered was allowed to react in 50 microliters of a reaction mixture [33 mM trisaminomethane-acetic acid buffer (pH 7.9), 66 mM potassium acetate, 10 mM magnesium acetate, 0.5 mM DTT, 0.01 % bovine serum albumin, 0.1 mM dNTP, and 10 units of T4 DNA polymerase] at 37°C for 5 minutes, and DNA was recovered by ethanol precipitation. This DNA was allowed to react in 50 microliters of a reaction mixture [66 mM Tris-HCl (pH 7.6), 6.6 mM $MgCl_2$, 10 mM DTT, 0.1 mM ATP, 100 units of T4 DNA ligase, and 0.1 ng/microliter pBamHI linker (loc cit)].at 12°C for 15 hours, and DNA was again recovered by ethanol precipitation. The DNA recovered was allowed to react in 50 microliters of a reaction mixture [10 mM Tris-HCl (pH 7.5), 10 mM $MgCl_2$, 100 mM NaCl, 6 mM 2-mercaptoethanol, and 20 units each of BamHI and SacI] at 37°C for 5 hours. After the reaction, the DNA recovered by ethanol precipitation was mixed with 0.5 microgram of pHSG398 DNA, which had been allowed to react in 20 microliters of a reaction mixture [10 mM Tris-HCl (pH 7.5), 10 mM $MgCl_2$, 100 mM NaCl, 6 mM 2-mercaptoethanol, and 10 units each of BamHI and SacI] at 37°C for 5 hours. The mixture was then allowed to react in 50 microliters of a reaction mixture [66 mM Tris-HCl (pH 7.6), 6.6 mM $MgCl_2$, 10 mM DTT, 0.1 mM ATP, and 100 units of T4 DNA ligase] at 16°C for 3 hours. By using 20 microliters of this reaction mixture, E. coli JM105 was transformed according to the calcium chloride method. From chloramphenicol-resistant, beta-galactosidase alpha-donor non-producing transformants, pHSG-3′-VLC was selected.

(3-a-3) Preparation of ORF DNA of human ventricular myosin light chain I

One microgram each of pUC-5′-VLC4 and pHSG-3′-VLC DNA was allowed to react separately in 50 microliters of a reaction mixture [100 mM Tris-HCl (pH 7.5), 7 mM $MgCl_2$, 50 mM NaCl, 7 mM 2-mercaptoethanol, 0.01 % bovine serum albumin, and 20 units each of EcoRI and SacI] at 37°C for 5 hours. After ethanol precipitation, both of the DNA fractions recovered were mixed together and allowed to react in 50 microliters of a reaction mixture [66 mM Tris-HCl (pH 7.6), 6.6 mM $MgCl_2$, 10 mM DTT, 0.1 mM ATP, and 100 units of T4 DNA ligase] at 16°C for 5 hours. By using 20 microliters of this reaction mixture, E. coli JM105 was transformed according to the calcium chloride method. From transformants exhibiting both chloramphenicol resistance and ampicillin resistance, pHU-VLC4 was selected. The pHU-VLC4 has a structure consisting of the 5′-region and 3′-region of human ventricular myosin light chain I, ligated via the SacI cleavage sites, and its ORF can be constructed as a BamHI fragment.

(3-b) Introduction of human ventricular myosin light chain I ORF DNA into pDR540

Five micrograms of pHU-VLC4 DNA was allowed to react in 200 microliters of a reaction mixture [10 mM Tris-HCl (pH 7.5), 10 mM $MgCl_2$, 100 mM NaCl, 6 mM 2-mercaptoethanol, and 30 units of BamHI] at 37°C for 5 hours. After the reaction, a BamHI DNA fragment containing human ventricular myosin light chain I ORF was prepared according to the method of McDonnell et al. (J. Mol. Biol., 110, 119 (1977)). Separately, 0.5 microgram of expression plasmid vector pDR540 (Proc. Natl. Acad. Sci., USA, 80, 21 (1983); available from Pharmacia) DNA was allowed to react in 50 microliters of a reaction mixture [10 mM Tris-HCl (pH 7.5), 10 mM $MgCl_2$, 100 mM NaCl, 6 mM 2-mercaptoethanol, and 10 units of BamHI] at 37°C for 5 hours, and DNA was recovered by ethanol precipitation. The DNA recovered was mixed with the previously prepared BamHI DNA fragment containing human ventricular myosin light chain I ORF, and the mixture was allowed to react in 50 microliters of a reaction mixture [66 mM Tris-HCl (pH 7.6), 6.6 mM $MgCl_2$, 10 mM DTT, 0.1 mM ATP, and 100 units of T4 DNA ligase] at 16°C for 5 hours. By using 20 microliters of this reaction mixture, E. coli JM105 was transformed according to the calcium chloride method. From the ampicillin-resistant transformants obtained, pDR-VLC4 was selected. The pDR-VLC4 is a modified pDR540 in which human ventricular myosin light chain I ORF is inserted in the coordinate direction for transcription at the BamHI cleavage site downstream of the tac promoter. The distance between the SD sequence downstream of the tac promoter of pDR540 and the translational start codon of human ventricular myosin light chain I is 12 bp.

(3-c) Expression of human ventricular myosin light chain I gene in E. coli

E. coli JM105 carrying pDR-VLC4 was cultivated at 37°C in 2 x YT medium (1.6 % polypeptone, 1 % yeast extract, 0.5 % NaCl) supplemented with 20 micrograms/ml ampicillin. When $OD_{650}$ reached 0.20, with an addition of isopropyl-beta-D-thiogalactopyranoside (IPTG) at the final concentration of 1 mM, the cultivation was further continued at 37°C for 1 hour. After the cultivation, 10 ml of the culture was taken, quenched and centrifuged at 8.000 x g for 10 minutes so as to prepare cells. The cells obtained were suspended in 1 ml of buffer solution [50 mM Tris-HCl (pH 7.8), 20 mM EDTA, 1 mM PMSF, and 50 micromols/liter 2-mercaptoethanol] and destroyed by ultrasonic treatment. The suspension was then centrifuged at 8.000 x g for 10.minutes to remove cell debris. The supernatant was used as a sample for the determination of the human cardiac myosin light chain according to the method of Yazaki et al (Japanese Patent Laid-Open Pub. No. 286752/1986). Furthermore, the protein concentration of the sample was determined by using a protein assay kit (Bio-Rad Laboratories). As a result, the production of 230 ng myosin/mg protein in the E. coli was confirmed.

(4) Extraction and purification of human ventricular myosin light chain I expressed in E. coli:

E. coli JM105 carrying pDR-VLC4 was cultivated in 5 ml of 2 x YT medium (loc cit) supplemented with ampicillin (20 micrograms/ml) at 37°C for 6 hours, further subcultured in 200 ml of 2 x YT medium at 37°C for 12 hours. Thereafter, 200 ml of the resultant culture was added to 10 liters of 2 x YT medium and cultivated at 37°C for 2 hours, and, with an addition of IPTG at the final concentration of 1 mM, the cultivation was further continued for 6 hours.

The resultant culture was quenched and centrifuged at 8.000 x g for 10 minutes to recover cells. The cells thus recovered were suspended in 800 ml of a buffer solution [50 mM Tris-HCl (pH 7.8), 20 mM EDTA, and 50 micromols/liter 2-mercaptoethanol, 1 mM PMSF] and destroyed by ultrasonic treatment. The suspension was then centrifuged at 10,000 x g for 10 minutes to recover the supernatant.

Separately, 120 mg of anti-ventricular-myosin-light chain-I monoclonal antibody MLM544 (Japanese Patent Laid-Open Pub. No. 286752/1986) was bound to 12 ml of Sepharose CL4B gel (Pharmacia) and, after blocking with 1 M ethanolamine, the gel was charged into a column, and washed with 120 ml of phosphate-buffered saline (PBS) [8 mM $Na_2HPO_4$, 2 mM $KH_2PO_4$, 137 mM NaCl, and 3 mM KCl (pH 7.4)] containing 5 M guanidine and then with 120 ml of PBS. To the column thus prepared was applied 688 ml of the E. coli extract recovered, and the column was washed with 120 ml of PBS. Elution was carried out with PBS containing 5 M guanidine-HCl. The eluate was dialyzed against PBS whereby 1.3 mg of recombinant human ventricular myosin light chain I was obtained. Analysis of this recombinant human ventricular myosin chain I by SDS-polyacrylamide gel electrophoresis reveal a single band.

**Claims**

1. Substantially pure recombinant human cardiac myosin and fragments thereof free from other proteins of human origin.

2. Recombinant human cardiac myosin and fragments thereof as set forth in claim 1, wherein the human cardiac myosin is a human cardiac myosin light chain.

3. Recombinant human cardiac myosin and fragments thereof as set forth in claim 1 or 2, wherein the human cardiac myosin is human ventricular myosin.

4. Recombinant human cardiac myosin and fragments thereof as set forth in claim 1 or 2, wherein the human cardiac myosin is human atrial myosin.

5. Recombinant human cardiac myosin and fragments thereof, having the amino acid sequence of the formula (I):

Met-Ala-Pro-Lys-Lys-Pro-Glu-Pro-Lys-Lys-X1-
X2-Ala-Lys-X3-Ala-Pro-X4-X3-Ala-Pro-Ala-Pro-
Ala-Pro-Z (I)

wherein X1 is Glu or Asp, X2 is Ala or Asp, X3 is Pro or Ala, X4 is Ala or Lys, and Z is the amino acid sequence of the formula (II) or (III) shown below:

Pro Pro Glu Pro Glu Arg Pro Lys Glu Val Glu
Phe Asp Ala Ser Lys Ile Lys Ile Glu Phe Thr
Pro Glu Gln Ile Glu Glu Phe Lys Glu Ala Phe
Met Leu Phe Asp Arg Thr Pro Lys Cys Glu Met
Lys Ile Thr Tyr Gly Gln Cys Gly Asp Val Leu
Arg Ala Leu Gly Gln Asn Pro Thr Gln Ala Glu
Val Leu Arg Val Leu Gly Lys Pro Arg Gln Glu
Glu Leu Asn Thr Lys Met Met Asp Phe Glu Thr

Phe Leu Pro Met Leu Gln His Ile Ser Lys Asn
Lys Asp Thr Gly Thr Tyr Glu Asp Phe Val Glu
Gly Leu Arg Val Phe Asp Lys Glu Gly Asn Gly
Thr Val Met Gly Ala Glu Leu Arg His Val Leu
Ala Thr Leu Gly Glu Arg Leu Thr Glu Asp Glu
Val Glu Lys Leu Met Ala Gly Gln Glu Asp Ser
Asn Gly Cys Ile Asn Tyr Glu Ala Phe Val Lys
His Ile Met Ser Ser (II)

Ala Pro Ala Pro Ala Pro Glu Ala Pro Lys Glu
Pro Ala Phe Asp Pro Lys Ser Val Lys Ile Asp
Phe Thr Ala Glu Gln Ile Glu Glu Phe Lys Glu
Ala Phe Ser Leu Phe Asp Arg Thr Pro Thr Gly
Glu Met Lys Ile Thr Tyr Gly Gln Cys Gly Asp
Val Leu Arg Ala Leu Gly Gln Asn Pro Thr Asn
Ala Glu Val Leu Arg Val Leu Gly Lys Pro Lys
Pro Glu Glu Met Asn Val Lys Met Leu Asp Phe
Glu Thr Phe Leu Pro Ile Leu Gln His Ile Ser
Arg Asn Lys Glu Gln Gly Thr Glu Asp Asp Phe
Val Glu Gly Leu Arg Val Phe Glu Lys Glu Ser
Asn Gly Thr Asp Met Gly Ala Glu Leu Arg His
Val Leu Ala Thr Leu Gly Glu Lys Met Thr Glu
Val Glu Val Glu Gln Leu Leu Ala Gly Gln Glu
Asp Ala Asn Gly Cys Ile Asn Tyr Glu Ala Phe
Val Lys His Ile Met Ser Gly (III)

6. Recombinant human cardiac myosin and fragments thereof as set forth in claim 5 represented by the formula (IV):

Met Ala Pro Lys Lys Pro Glu Pro Lys Lys Asp
Asp Ala Lys Ala Ala Pro Lys Ala Ala Pro Ala
Pro Ala Pro Pro Pro Glu Pro Glu Arg Pro Lys
Glu Val Glu Phe Asp Ala Ser Lys Ile Lys Ile
Glu Phe Thr Pro Glu Gln Ile Glu Glu Phe Lys
Glu Ala Phe Met Leu Phe Asp Arg Thr Pro Lys
Cys Glu Met Lys Ile Thr Tyr Gly Gln Cys Gly
Asp Val Leu Arg Ala Leu Gly Gln Asn Pro Thr
Gln Ala Glu Val Leu Arg Val Leu Gly Lys Pro
Arg Gln Glu Glu Leu Asn Thr Lys Met Met Asp
Phe Glu Thr Phe Leu Pro Met Leu Gln His Ile
Ser Lys Asn Lys Asp Thr Gly Thr Tyr Glu Asp
Phe Val Glu Gly Leu Arg Val Phe Asp Lys Glu
Gly Asn Gly Thr Val Met Gly Ala Glu Leu Arg
His Val Leu Ala Thr Leu Gly Glu Arg Leu Thr
Glu Asp Glu Val Glu Lys Leu Met Ala Gly Gln
Glu Asp Ser Asn Gly Cys Ile Asn Tyr Glu Ala
Phe Val Lys His Ile Met Ser Ser (IV)

7. Recombinant human cardiac myosin and fragments thereof as set forth in claim 5 represented by the formula (V):

Met Ala Pro Lys Lys Pro Glu Pro Lys Lys Glu

Ala Ala Lys Pro Ala Pro Ala Pro Ala Pro Ala

Pro Ala Pro Ala Pro Ala Pro Ala Pro Glu Ala

Pro Lys Glu Pro Ala Phe Asp Pro Lys Ser Val

Lys Ile Asp Phe Thr Ala Glu Gln Ile Glu Glu

Phe Lys Glu Ala Phe Ser Leu Phe Asp Arg Thr

Pro Thr Gly Glu Met Lys Ile Thr Tyr Gly Gln

Cys Gly Asp Val Leu Arg Ala Leu Gly Gln Asn

Pro Thr Asn Ala Glu Val Leu Arg Val Leu Gly

Lys Pro Lys Pro Glu Glu Met Asn Val Lys Met

Leu Asp Phe Glu Thr Phe Leu Pro Ile Leu Gln

His Ile Ser Arg Asn Lys Glu Gln Gly Thr Glu

Asp Asp Phe Val Glu Gly Leu Arg Val Phe Glu

Lys Glu Ser Asn Gly Thr Asp Met Gly Ala Glu

Leu Arg His Val Leu Ala Thr Leu Gly Glu Lys

Met Thr Glu Val Glu Val Glu Gln Leu Leu Ala

Gly Gln Glu Asp Ala Asn Gly Cys Ile Asn Tyr

Glu Ala Phe Val Lys His Ile Met Ser Gly

( V )

8. A DNA sequence coding for human cardiac myosin and DNA fragments derived therefrom.

9. A DNA sequence coding for human cardiac myosin and DNA fragments derived therefrom, said DNA sequence being represented by the following formula (XI):

Y4-Y9-Y7-Y14-Y14-Y7-Y16-Y7-Y14-Y14-W1-W2-Y9-

Y14-W3-Y9-Y7-W4-W3-Y9-Y7-Y9-Y7-Y9-Y7-X

( XI )

wherein

Y4 is ATG coding for Met,

Y7 is CCT, CCC, CCA or CCG coding for Pro,

Y9 is GCT, GCC, GCA or GCG coding for Ala,
Y14 is AAA or AAG coding for Lys,
Y16 is GAA or GAG coding for Glu,
W1 is GAA or GAG coding for Glu or GAT or GAC coding for Asp,
W2 is GCT, GCC, GCA or GCG coding for Ala or GAT or GAC coding for Asp,
W3 is CCT, CCC, CCA or CCG coding for Pro or GCT, GCC, GCA or GCG coding for Ala,
W4 is GCT, GCC, GCA or GCG coding for Ala or AAA or AAG coding for Lys,
and X is the DNA sequence of the following formula (XII) or (XIII):

Y7-Y7-Y16-Y7-Y16-Y19-Y7-Y14-Y16-Y5-Y16-Y1-

Y15-Y9-Y6-Y14-Y3-Y14-Y3-Y16-Y1-Y8-Y7-Y16-

Y12-Y3-Y16-Y16-Y1-Y14-Y16-Y9-Y1-Y4-Y2-Y1-

Y15-Y19-Y8-Y7-Y14-Y17-Y16-Y4-Y14-Y3-Y8-Y10-

Y20-Y12-Y17-Y20-Y15-Y5-Y2-Y19-Y9-Y2-Y20-Y12-

Y13-Y7-Y8-Y12-Y9-Y16-Y5-Y2-Y19-Y5-Y2-Y20-

Y14-Y7-Y19-Y12-Y16-Y16-Y2-Y13-Y8-Y14-Y4-Y4-
Y15-Y1-Y16-Y8-Y1-Y2-Y7-Y4-Y2-Y12-Y11-Y3-Y6-
Y14-Y13-Y14-Y15-Y8-Y20-Y8-Y10-Y16-Y15-Y1-Y5-
Y16-Y20-Y2-Y19-Y5-Y1-Y15-Y14-Y16-Y20-Y13-
Y20-Y8-Y5-Y4-Y20-Y9-Y16-Y2-Y19-Y11-Y5-Y2-Y9-
Y8-Y2-Y20-Y16-Y19-Y2-Y8-Y16-Y15-Y16-Y5-Y16-
Y14-Y2-Y4-Y9-Y20-Y12-Y16-Y15-Y6-Y13-Y20-Y17-
Y3-Y13-Y10-Y16-Y9-Y1-Y5-Y14-Y11-Y3-Y4-Y6-Y6-
Z (XII)

Y9-Y7-Y9-Y7-Y9-Y7-Y16-Y9-Y7-Y14-Y16-Y7-Y9-
Y1-Y15-Y7-Y14-Y6-Y5-Y14-Y3-Y15-Y1-Y8-Y9-Y16-
Y12-Y3-Y16-Y16-Y1-Y14-Y16-Y9-Y1-Y6-Y2-Y1-
Y15-Y19-Y8-Y7-Y8-Y20-Y16-Y4-Y14-Y3-Y8-Y10-
Y20-Y12-Y17-Y20-Y15-Y5-Y2-Y19-Y9-Y2-Y20-Y12-
Y13-Y7-Y8-Y13-Y9-Y16-Y5-Y2-Y19-Y5-Y2-Y20-
Y14-Y7-Y14-Y7-Y16-Y16-Y4-Y13-Y5-Y14-Y4-Y2-
Y15-Y1-Y16-Y8-Y1-Y2-Y7-Y3-Y2-Y12-Y11-Y3-Y6-
Y19-Y13-Y14-Y16-Y12-Y20-Y8-Y16-Y15-Y15-Y1-
Y5-Y16-Y20-Y2-Y19-Y5-Y1-Y16-Y14-Y16-Y6-Y13-
Y20-Y8-Y15-Y4-Y20-Y9-Y16-Y2-Y19-Y11-Y5-Y2-
Y9-Y8-Y2-Y20-Y16-Y14-Y4-Y8-Y16-Y5-Y16-Y5-
Y16-Y12-Y2-Y2-Y9-Y20-Y12-Y16-Y15-Y9-Y13-Y20-
Y17-Y3-Y13-Y10-Y16-Y9-Y1-Y5-Y14-Y11-Y3-Y4-
Y6-Y20-Z (XI)

wherein
Y1 is TTT or TTC coding for Phe,
Y2 is TTA, TTG, CTT, CTC, CTA or CTG coding for Leu,
Y3 is ATT, ATC or ATA coding for Ile,

Y5 is GTT, GTC, GTA or GTG coding for Val,
Y6 is TCT, TCC, TCA, TCG, AGT or AGC coding for Ser,
Y8 is ACT, ACC, ACA or ACG coding for Thr,
Y10 is TAT or TAC coding for Tyr,
Y11 is CAT or CAC coding for His,
Y12 is CAA or CAG coding for Gln,
Y13 is AAT or AAC coding for Asn,
Y15 is GAT or GAC coding for Asp,
Y17 is TGT or TGC coding for Cys,
Y18 is TGG coding for Trp,
Y19 is CGT, CGC, CGA, CGG, AGA or AGG coding for Arg,
Y20 is GGT, GGC, GGA or GGG coding for Gly, and
Z is a stop codon which is TAA, TAG or TGA.

10. A DNA sequence and DNA fragments derived therefrom as set forth in claim 9, said DNA sequence being represented by the following formula (XIV):

```
ATG GCC CCC AAA AAG CCA GAG CCC AAG AAG GAT
GAT GCC AAG GCA GCC CCC AAG GCA GCT CCA GCT
CCC GCA CCT CCC CCT GAG CCT GAG CGC CCT AAG
GAG GTC GAG TTT GAT GCT TCC AAG ATC AAG ATT
GAG TTC ACA CCT GAG CAG ATT GAA GAG TTC AAG
GAA GCC TTC ATG CTG TTC GAC CGC ACA CCC AAG
TGT GAG ATG AAG ATC ACC TAC GGG CAG TGT GGG
GAT GTC CTG CGG GCG CTG GGC CAG AAC CCC ACA
CAG GCA GAA GTG CTC CGT GTC CTG GGG AAG CCA

AGA CAG GAA GAG CTC AAT ACC AAG ATG ATG GAC
TTT GAA ACT TTC CTG CCT ATG CTC CAG CAC ATT
TCC AAG AAC AAG GAC ACA GGC ACC TAT GAG GAC
TTC GTG GAG GGG CTG CGG GTC TTC GAC AAG GAG
GGC AAT GGC ACT GTC ATG GGT GCT GAG CTT CGC
CAC GTG CTG GCC ACG CTG GGT GAG AGG CTG ACA
GAA GAC GAA GTG GAG AAG TTG ATG GCT GGG CAA
GAG GAC TCC AAT GGC TGC ATC AAC TAT GAA GCA
TTT GTG AAG CAC ATC ATG TCC AGC Z        (XIV)
```

wherein Z is a stop codon which is TAA, TAG or TGA.

11. A DNA sequence and DNA fragments derived therefrom as set forth in claim 9, said DNA sequence being represented by the following formula (XV):

ATG GCT CCC AAG AAG CCT GAG CCT AAG AAG GAG
GCA GCC AAG CCA GCT CCA GCT CCA GCT CCA GCC
CCT GCA CCA GCC CCT GCC CCA GCT CCT GAG GCT
CCC AAG GAA CCT GCC TTT GAC CCC AAG AGT GTA
AAG ATA GAC TTC ACT GCC GAG CAG ATT GAA GAG
TTA AAA GAG GCC TTT TCA TTG TTT GAC CGG ACC
CCG ACT GGA GAG ATG AAG ATC ACC TAC GGC CAG
TGC GGG GAT GTA CTG CGG GCC CTG GGC CAG AAC
CCT ACC AAT GCC GAG GTG CTG CGT GTG CTG GGC

AAG CCC AAG CCT GAA GAG ATG AAT GTC AAG ATG
CTG GAC TTT GAG ACG TTC TTG CCC ATC CTG CAG
CAC ATT TCC CGC AAC AAG GAG CAG GGC ACC TAT
GAG GAC TTC GTG GAG GGC CTG CGT GTC TTT GAG
AAG GAG AGC AAT GGC ACG GAC ATG GGT GCT GAG
CTT CGG CAC GTC CTT GCC ACC CTG GGA GAG AAG
ATG ACT GAG GCT GAA GTG GAG CAG CTG TTG GCT
GGG CAA GAG GAT GCC AAT GGC TGC ATC AAT TAT
GAA GCC TTT GTC AAG CAC ATC ATG TCA GGG Z

(XV)

wherein z is a stop codon which is TAA, TAG or TGA.

12. A method of producing recombinant human cardiac myosin comprising the steps of:

(a) isolating a cDNA clone coding for human cardiac myosin,
(b) introducing the cDNA obtained in the step (a) into an expression vector,
(c) transforming a host cell by the expression vector obtained in the step (b),
(d) culturing the transformants obtained in the step (c), and

(e) extracting recombinant human cardiac myosin from the culture obtained in the culturing step (d) and purifying the same.

13. A method of producing recombinant human cardiac myosin as set forth in claim 12, wherein said cDNA clone coding for human cardiac myosin is prepared by isolating messenger RNA (mRNA) from human myocardial cells, constructing a library for DNA complementary to said mRNA (cDNA), and selecting the cDNA clone coding for human cardiac myosin from said cDNA library using a probe.

14. An expression vector containing a cDNA coding for human cardiac myosin.

15. A host cell transformed by an expression vector containing a cDNA coding for human cardiac myosin.

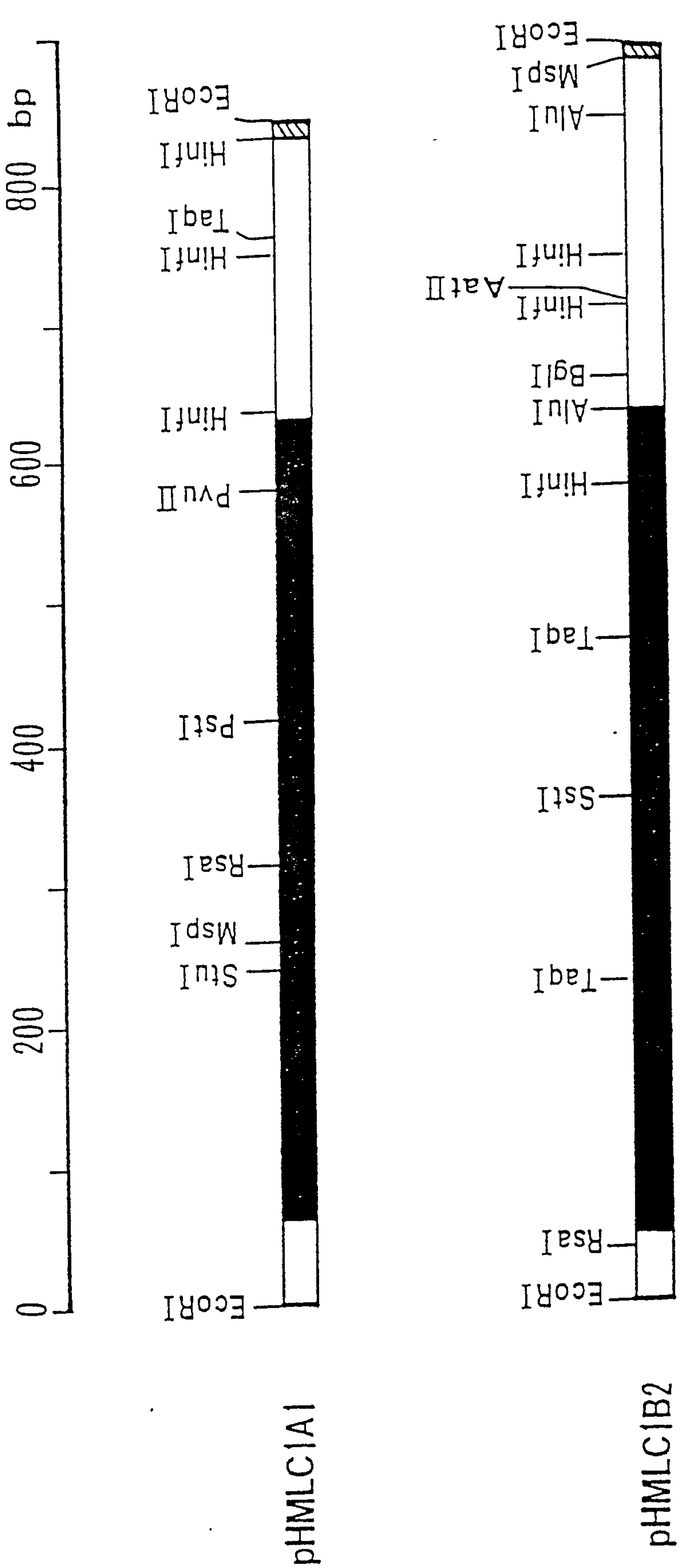
0
200
400
600
800
bp
EcoRI
HinfI
TaqI
HinfI
HinfI
PvuII
PstI
RsaI
MspI
StuI
EcoRI
pHMLC1A1
EcoRI
MspI
AluI
HinfI
AatII
HinfI
BglI
AluI
HinfI
TaqI
SstI
TaqI
RsaI
EcoRI
pHMLC1B2

FIG. I

```
CCTCGGTCTCTCGGCTTTCTTCTTAGATCACTCCTCTGCCAAAGATCCCAACAAGGACTAAC  -1

ATG GCT CCC AAG AAG CCT GAG CCT AAG AAG GAG GCA GCC AAG CCA GCT CCA GCT CCA GCT  60
Met Ala Pro Lys Lys Pro Glu Pro Lys Lys Glu Ala Ala Lys Pro Ala Pro Ala Pro Ala

CCA GCC CCT GCA CCA GCC CCT GCC CCA GCT CCT GAG GCT CCC AAG GAA CCT GCC TTT GAC 120
Pro Ala Pro Ala Pro Ala Pro Ala Pro Ala Pro Glu Ala Pro Lys Glu Pro Ala Phe Asp

CCC AAG AGT GTA AAG ATA GAC TTC ACT GCC GAG CAG ATT GAA GAG TTA AAA GAG GCC TTT 180
Pro Lys Ser Val Lys Ile Asp Phe Thr Ala Glu Gln Ile Glu Glu Phe Lys Glu Ala Phe

TCA TTG TTT GAC CGG ACC CCG ACT GGA GAG ATG AAG ATC ACC TAC GGC CAG TGC GGG GAT 240
Ser Leu Phe Asp Arg Thr Pro Thr Gly Glu Met Lys Ile Thr Tyr Gly Gln Cys Gly Asp

GTA CTG CGG GCC CTG GGC CAG AAC CCT ACC AAT GCC GAG GTG CTG CGT GTG CTG GGC AAG 300
Val Leu Arg Ala Leu Gly Gln Asn Pro Thr Asn Ala Glu Val Leu Arg Val Leu Gly Lys

CCC AAG CCT GAA GAG ATG AAT GTC AAG ATG CTG GAC TTT GAG ACG TTC TTG CCC ATC CTG 360
Pro Lys Pro Glu Glu Met Asn Val Lys Met Leu Asp Phe Glu Thr Phe Leu Pro Ile Leu

CAG CAC ATT TCC CGC AAC AAG GAG CAG GGC ACC TAT GAG GAC TTC GTG GAG GGC CTG CGT 420
Gln His Ile Ser Arg Asn Lys Glu Gln Gly Thr Glu Asp Asp Phe Val Glu Gly Leu Arg

GTC TTT GAG AAG GAG AGC AAT GGC ACG GAC ATG GGT GCT GAG CTT CGG CAC GTC CTT GCC 480
Val Phe Glu Lys Glu Ser Asn Gly Thr Asp Met Gly Ala Glu Leu Arg His Val Leu Ala

ACC CTG GGA GAG AAG ATG ACT GAG GCT GAA GTG GAG CAG CTG TTG GCT GGG CAA GAG GAT 540
Thr Leu Gly Glu Lys Met Thr Glu Val Glu Val Glu Gln Leu Leu Ala Gly Gln Glu Asp

GCC AAT GGC TGC ATC AAT TAT GAA GCC TTT GTC AAG CAC ATC ATG TCA GGG TGA AGCAGAG 601
Ala Asn Gly Cys Ile Asn Tyr Glu Ala Phe Val Lys His Ile Met Ser Gly ***

TCTTCCAGGTGCCTGGCCCTTGGCTTTAGCCATACCAGGGTGAGTTAAAGAGAGAGGCCCCGGCTGGGTGAGCTGAGAT 680
GGAGTCCTCGACTTATTACCACACCACTGCCCCAAGGACCTTACAGGCCCTCCCTGTTAATAAACAGGCTGGGCTCTGG 759
GATTCTG(A)n
```

FIG.2 A

```
                          CTGCTTTCTGCATTCTTCTCTCCACATGCCTCTCTGTACTTACAGCCCCA  -1

ATG GCC CCC AAA AAG CCA GAG CCC AAG AAG GAT GAT GCC AAG GCA GCC CCC AAG GCA GCT   60
Met Ala Pro Lys Lys Pro Glu Pro Lys Lys Asp Asp Ala Lys Ala Ala Pro Lys Ala Ala

CCA GCT CCC GCA CCT CCC CCT GAG CCT GAG CGC CCT AAG GAG GTC GAG TTT GAT GCT TCC  120
Pro Ala Pro Ala Pro Pro Pro Glu Pro Glu Arg Pro Lys Glu Val Glu Phe Asp Ala Ser

AAG ATC AAG ATT GAG TTC ACA CCT GAG CAG ATT GAA GAG TTC AAG GAA GCC TTC ATG CTG  180
Lys Ile Lys Ile Glu Phe Thr Pro Glu Gln Ile Glu Glu Phe Lys Glu Ala Phe Met Leu

TTC GAC CGC ACA CCC AAG TGT GAG ATG AAG ATC ACC TAC GGG CAG TGT GGG GAT GTC CTG  240
Phe Asp Arg Thr Pro Lys Cys Glu Met Lys Ile Thr Tyr Gly Gln Cys Gly Asp Val Leu

CGG GCG CTG GGC CAG AAC CCC ACA CAG GCA GAA GTG CTC CGT GTC CTG GGG AAG CCA AGA  300
Arg Ala Leu Gly Gln Asn Pro Thr Gln Ala Glu Val Leu Arg Val Leu Gly Lys Pro Arg

CAG GAA GAG CTC AAT ACC AAG ATG ATG GAC TTT GAA ACT TTC CTG CCT ATG CTC CAG CAC  360
Gln Glu Glu Leu Asn Thr Lys Met Met Asp Phe Glu Thr Phe Leu Pro Met Leu Gln His

ATT TCC AAG AAC AAG GAC ACA GGC ACC TAT GAG GAC TTC GTG GAG GGG CTG CGG GTC TTC  420
Ile Ser Lys Asn Lys Asp Thr Gly Thr Tyr Glu Asp Phe Val Glu Gly Leu Arg Val Phe

GAC AAG GAG GGC AAT GGC ACT GTC ATG GGT GCT GAG CTT CGC CAC GTG CTG GCC ACG CTG  480
Asp Lys Glu Gly Asn Gly Thr Val Met Gly Ala Glu Leu Arg His Val Leu Ala Thr Leu

GGT GAG AGG CTG ACA GAA GAC GAA GTG GAG AAG TTG ATG GCT GGG CAA GAG GAC TCC AAT  540
Gly Glu Arg Leu Thr Glu Asp Glu Val Glu Lys Leu Met Ala Gly Gln Glu Asp Ser Asn

GGC TGC ATC AAC TAT GAA GCA TTT GTG AAG CAC ATC ATG TCC AGC TAA ACCTCGTGCCCAGAA  604
Gly Cys Ile Asn Tyr Glu Ala Phe Val Lys His Ile Met Ser Ser ***

GCCAGGAAGGCTGTGCAGGACGTCTCATCTCCCATGTGTGATGCTGACACCAAGCGGCCTGGAGTCGTGGGAAGGAGGG  683
GAGCCTTACCAAGACTCCTGCAAAAACCCTTGGACCTCTCCACGTGGTTGCCGTCTCTGGCCCCTCTCAGGCTGTGCTT  762
ACCTGTGACAGCAGCTCTGTCCCCACCCCATGGGCCTCATGAATAAAGGCTTCTTGCCGGC(A)n
```

FIG. 2 B

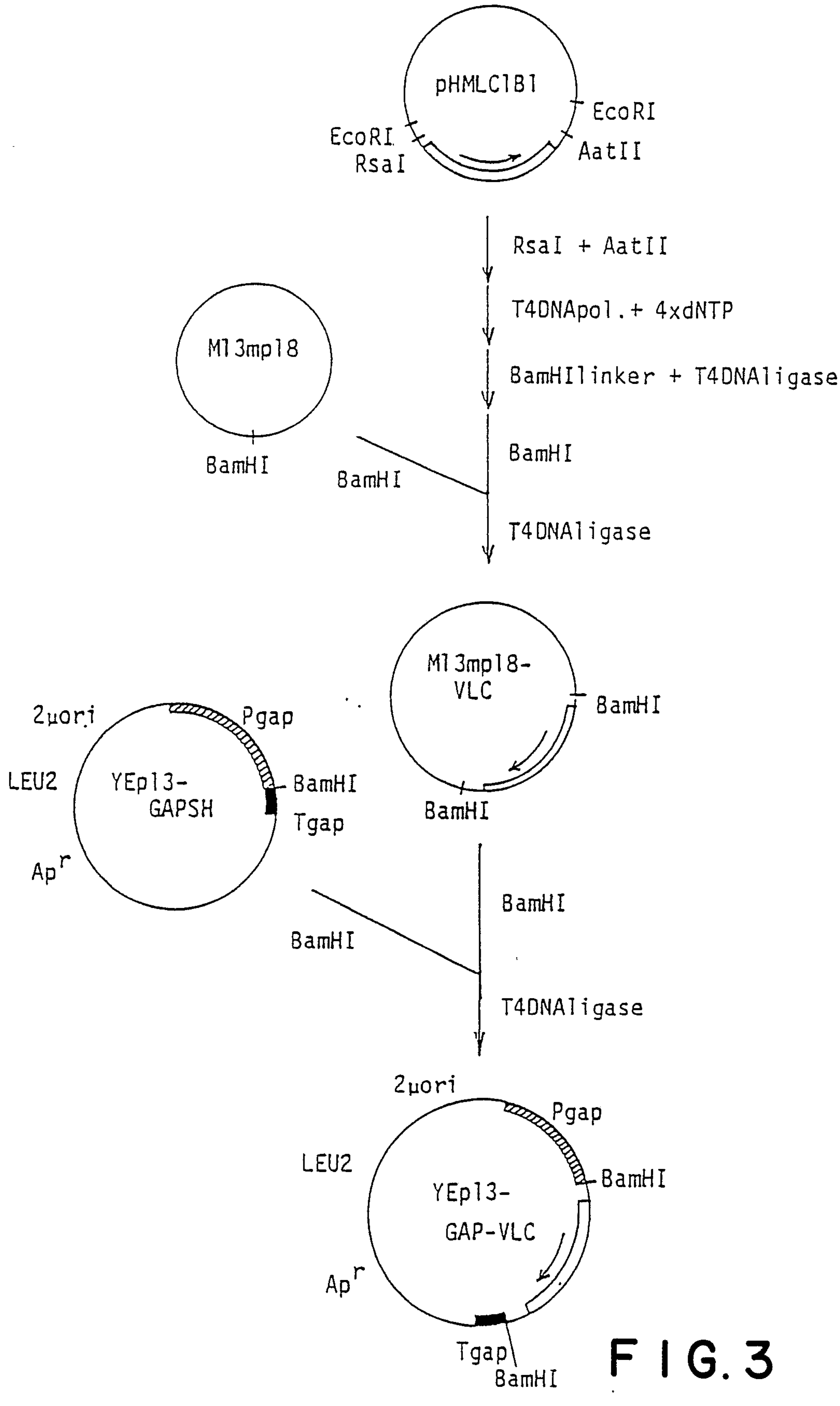
pHMLC1B1
EcoRI
EcoRI
RsaI
AatII
RsaI + AatII
T4DNApol.+ 4xdNTP
M13mp18
BamHIlinker + T4DNAligase
BamHI
BamHI
BamHI
T4DNAligase
M13mp18-
VLC
BamHI
BamHI
2μori
Pgap
LEU2
YEp13-
GAPSH
BamHI
Tgap
Apr
BamHI
BamHI
T4DNAligase
2μori
Pgap
LEU2
BamHI
YEp13-
GAP-VLC
Apr
Tgap
BamHI

FIG. 3

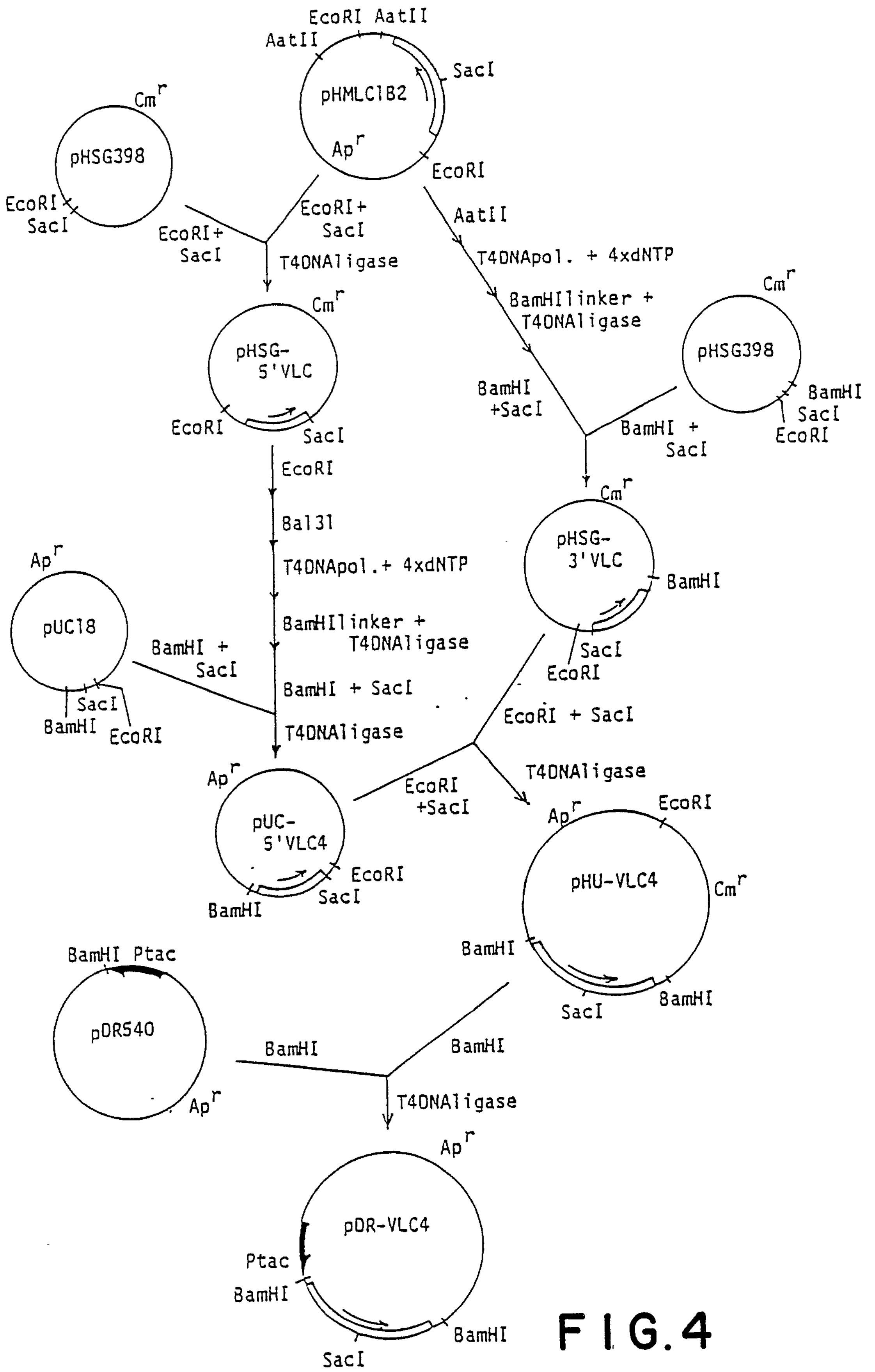

EcoRI AatII
AatII
SacI
pHMLC1B2
Apr
EcoRI
Cmr
pHSG398
EcoRI
SacI
EcoRI+
SacI
EcoRI+
SacI
T4DNAligase
AatII
T4DNApol. + 4xdNTP
BamHIlinker +
T4DNAligase
Cmr
pHSG398
BamHI
SacI
EcoRI
BamHI
+SacI
BamHI +
SacI
Cmr
pHSG-
5'VLC
EcoRI
SacI
EcoRI
Bal31
T4DNApol.+ 4xdNTP
BamHIlinker +
T4DNAligase
BamHI + SacI
T4DNAligase
Cmr
pHSG-
3'VLC
BamHI
SacI
EcoRI
Apr
pUC18
SacI
BamHI
EcoRI
BamHI +
SacI
EcoRI + SacI
T4DNAligase
Apr
pUC-
5'VLC4
EcoRI
SacI
BamHI
EcoRI
+SacI
Apr
EcoRI
pHU-VLC4
Cmr
BamHI
SacI
BamHI
BamHI Ptac
pDR540
Apr
BamHI
BamHI
T4DNAligase
Apr
pDR-VLC4
Ptac
BamHI
SacI
BamHI


FIG.4

European Patent Office

# EUROPEAN SEARCH REPORT

Application Number

EP 89 10 4607

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | CHEMICAL ABSTRACTS vol. 109, no. 13, 26 September 1988, abstract no. 105686a E. Hoffmann et al. "Molecular cloning and complete nucleotide sequence of a human ventricular myosin light chain"; & Nucleic Acids Res. 1988, vol. 16 (5, Pt.B), page 2353 --- | 1-3,5-6,8-10,12-15 | C 12 N 15/00<br>C 12 P 21/02<br>C 07 K 13/00 |
| D,X | CIRCULATION RESEARCH vol. 50, no. 2, February 1982, New York, USA; Catherine Klotz et al. "Comparative Sequence of Myosin Light Chains from Normal and Hypertrophied Human Hearts", pages 201-209 * whole document, in particular Table 1 * --- | 1-3,5-6 | |
| D,Y | EUROPEAN JOURNAL OF BIOCHEMISTRY vol. 148, 1985; Berlin, WG; Neil D. Vincent et al. "Isolation of cardiac myosin light-chain isotypes by chromatofocusing. Comparison of human cardiac atrial light-chain 1 and foetal ventricular light-chain 1", pages 135-143 * whole document, specially page 140 and page 142, column 1 * --- | 1-15 | **TECHNICAL FIELDS SEARCHED (Int. Cl.4)**<br>C 12 N 15/00<br>C 12 P 21/02<br>C 12 P 21/00 |
| D,Y | NUCLEIC ACIDS RESEARCH vol. 10, no. 19, 1982; London, GB; Yo-ichi Nabeshima et al. "Molicular cloning and nucleotide sequences of the complemantary DNAs to chicken skeletal muscle myosin two alkali light chain mRNAs", pages 6099-6110 * pages 6100 - 6102 * --- -/- | 1-15 | |

The present search report has been drawn up for all claims

| Place of search | Date of completion of the search | Examiner |
|---|---|---|
| BERLIN | 07-06-1989 | JULIA P. |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)

European Patent Office

# EUROPEAN SEARCH REPORT

Application Number

EP 89 10 4607

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,X | JOURNAL OF BIOLOGICAL CHEMISTRY vol. 263, no. 27, 25 September 1988, Baltimore, MD, USA; Masahiko Kurabayashi et al. "Molecular Cloning and Characterization of Human Atrial and Ventricular Myosin Alkali Light Chain cDNA Clones", pages 13930-13936 * whole document * | 1-15 | |
| X | BIOSIS DATABASE abstract no. 34041654; M. Kurabayashi et al. "Molecular Cloning of mRNA Sequences for Atrial and Ventriclar Myosin Light Chain 1 from Human Heart and Characterization of Their Genomic Organizations"; & Circulation (US) 1987, vol. 76, no. 4, part 2, page IV-475 | 1-15 | |
| A | BIOSIS DATABASE abstract no. 30085835; I. Balazs et al. "Chromosomal mapping and characterization of human DNA sequences homologous to cardiac myosin light chain gene"; & Cytogenetics and Cell Genetics 1985, vol. 40, no. 1-4, pages 574-575 | 1-15 | TECHNICAL FIELDS SEARCHED (Int. Cl.4) |

The present search report has been drawn up for all claims

| Place of search | Date of completion of the search | Examiner |
|---|---|---|
| BERLIN | 07-06-1989 | JULIA P. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)